# EUROPÄISCHE PATENTANMELDUNG

(11) **EP 3 443 967 A1**
(43) Veröffentlichungstag der Anmeldung: **20.02.2019**
(21) Anmeldenummer: 17186727.8
(22) Anmeldetag: 17.08.2017
(51) Int. Cl.: A61K 35/38

(54) **VERFAHREN ZUR EINLAGERUNG DER BAKTERIENFLORA DES DARMS EINES LEBEWESENS, INSBESONDERE EINES MENSCHEN**

(71) Anmelder: Labor L + S Aktiengesellschaft, 97708 Bad Bocklet-Großenbrach (DE)
(72) Erfinder: BALLES, Jürgen, 97723 Oberthulba (DE); RÜFFER, Andreas, 97708 Bad Bocklet (DE)
(74) Vertreter: Albrecht, Ralf

(57) **Zusammenfassung**

Die Erfindung betrifft ein Verfahren zur Einlagerung der Bakterienflora des Darms eines Lebewesens, insbesondere eines Menschen, bei welchem die folgenden Schritte durchgeführt werden:
a) der Stuhl eines Spenders wird in einer physiologischen Pufferlösung, insbesondere einer physiologischen Kochsalzlösung, homogenisiert,
b) aus dem entstehenden Homogenisat wird durch wenigstens einen Filtrationsschritt und/oder wenigstens einen Zentrifugierungsschritt ein Bakterienkonzentrat gewonnen und
c) das Bakterienkonzentrat wird konserviert und eingelagert.

## Beschreibung

Die vorliegende Erfindung betrifft ein Verfahren zur Einlagerung der Bakterienflora des Darms eines Lebewesens, insbesondere eines Menschen. Im menschlichen Darm leben viele Billionen Bakterien. Mehrere hundert verschiedene Spezies besiedeln insbesondere den Dickdarm und bilden das sog. Mikrobiom. Diese Bakterien sind nicht nur für das menschliche Immunsystem relevant, sondern auch für die Verdauung von Ballaststoffen verantwortlich. Sie produzieren Enzyme, welche diese für uns sonst unverwertbaren Nahrungsbestandteile aufspalten. Außerdem wehren sie Krankheitserreger ab und produzieren Vitamine.

Es ist bekannt, dass die Zusammensetzung des Mikrobioms von vielen unterschiedlichen Faktoren, u.a. auch von der Lebensweise jedes einzelnen Menschen abhängt. Verschiebungen und Ungleichgewichte in der Zusammensetzung der Darmflora können Allergien, die Neigung zur Fettsucht und andere Krankheiten verursachen. Vor diesem Hintergrund ist eine gesunde, ausgewogene Darmflora wichtig für die Gesundheit eines jeden Menschen.

Insofern erfahren der Darm und seine Bakterienflora in den letzten Jahren ein immer größeres Interesse, und zwar insbesondere im Rahmen der Beseitigung chronischer Darmentzündungen. Bei einem Befall des Darms mit Bakterieninfektion, beispielsweise Entzündungen der Darmwand, die von Giften des Bakteriums Clostridium difficile herrühren, erfolgt die Behandlung regelmäßig mit Antibiotika. Ein Problem besteht jedoch darin, dass die eingesetzten Antibiotika keinen Unterschied zwischen guten und für die Gesundheit unverzichtbaren Bakterien im Darm und denen, durch welche die zu behandelnde Entzündung verursacht wurden, machen. Die Folge ist ein allgemeines Bakteriensterben im Darm der Patienten, und nicht selten breiten sich auf den freien Plätzen der abgestorbenen guten Bakterien die schädlichen Chlostridien aus. Die Folge ist häufig eine Entzündung des Darms, bis hin zur sog. pseudomembranösen Kolitis, mit einhergehenden hartnäckigen Durchfällen. Nach einer längeren Behandlung mit Antibiotika wird deshalb regelmäßig ein Neuaufbau der Darmflora medikamentös und/oder mit probiotischen Lebensmitteln begleitet.

Eine seit einigen Jahren eingesetzte alternative Behandlung von symptomatischen Infektionen mit Clostridium difficile, die seit Mitte 2016 auch in Deutschland zugelassen ist, besteht in einer Stuhltransplantation, bei welcher Stuhl von gesunden Spendern in den Darm von Kranken appliziert wird.

Die Stuhltransplantation, d.h. die Übertragung der Darmflora von einer auf eine andere Person aus therapeutischen Gründen wird in der Literatur mit unterschiedlichen Begriffen bezeichnet. In der angelsächsischen Literatur wird häufig der Begriff "fecal microbiota-transplantation" verwendet, der so viel wie "fäkale Darmfloratransplantation" oder "Stuhlbakterientransplantation" bedeutet. Auch ist es üblich die Vorgehensweise einfach zu beschreiben.

Versucht wurde die Übertragung der Darmflora bei der Clostridium-difficile-assoziierten Kolitis. Der Ansatz hierbei besteht darin, dass eine geschädigte Darmflora Rückfälle stark begünstigt, da sich Clostridium-Difficile-Keime mangels fehlender Konkurrenz physiologischer Darmkeime ungehindert vermehren können. Die "Biodiversität" des Stuhls ist bei einer Antibiotika-assoziierten Kolitis im Vergleich zu normalem Stuhl deutlich reduziert.

Im Rahmen der Stuhlbakterientransplantation wird dem Patienten über eine Darmspiegelung der Stuhl eines gesunden Spenders injiziert. Der gewonnene Stuhl wird mit Wasser oder Kochsalzlösung verdünnt, gefiltert und dann entweder über ein Klysma, im Rahmen einer Koloskopie oder über eine Dünndarmsonde in den Darm gegeben. Hier siedeln sich die übertragenen Bakterien im Darm des Empfängers an.

Ein Problem bei der Stuhltransplantation besteht darin, dass der Stuhl eines geeigneten Spenders bei der Transplantation zur Verfügung stehen muss. Zum Teil ist es möglich, den Stuhl eines Spenders unmittelbar vor der Transplantation zu gewinnen und für die Transplantation vorzubereiten. Dies gilt insbesondere wenn beispielsweise der Stuhl von den Kindern eines Patienten stammt. Dies ist jedoch nicht immer möglich.

Vor diesem Hintergrund liegt die Aufgabe der vorliegenden Erfindung darin, ein Verfahren anzugeben, mit dem der Stuhl eines Spenders für eine Transplantation auch längerfristig eingelagert werden kann.

Erfindungsgemäß ist diese Aufgabe gelöst durch ein Verfahren, bei welchem die folgenden Schritte durchgeführt werden:
a) der Stuhl eines Spenders wird in einer physiologischen Pufferlösung, insbesondere einer physiologischen Kochsalzlösung, homogenisiert,
b) aus dem entstehenden Homogenisat wird durch wenigstens einen Filtrationsschritt und/oder wenigstens einen Zentrifugierungsschritt ein Bakterienkonzentrat gewonnen und
c) das Bakterienkonzentrat wird konserviert und eingelagert.

Der Erfindung liegt somit die Überlegung zugrunde, aus dem Stuhl eines Spenders sämtliche Bakterien zu isolieren, diese zu konservieren und sie für eine eigene spätere Verwendung insbesondere im Sinne einer potentiellen "Eigen-Flora-Transplantation" einzulagern. Eine solche Einlagerung ist beispielsweise vor Operationen, antibiotischen Behandlungen oder anderen Eingriffen möglich, die voraussichtlich zu einer Schädigung der Darmflora führen. Mit dem eingelagerten Stuhl kann der Darm mit der eigenen Flora wieder "regeneriert" werden. Ebenso können Eltern die Darmbakterien ihrer Kinder einlagern lassen, damit diese später als "Darmflora-Backup" genutzt werden können (vergleichbar mit der Einlagerung von Nabelschnurblut).

Für den Nutzer liegen die Hauptvorteile darin, dass die eigene Darmflora eingesetzt werden kann, also keine fremden Spender erforderlich sind und desweiteren nicht der Stuhl, sondern nur die daraus isolierten Bakterien für die Behandlung eingesetzt werden. Zur Haltbarmachung sieht die Erfindung vor, dass der Stuhl eines Spenders zunächst in einer physiologischen Pufferlösung, insbesondere in einer sterilen physiologischen Kochsalzlösung homogenisiert wird. Aus dem entstehenden Homogenisat wird anschließend durch wenigstens einen Filtrationsschritt und/oder wenigstens einen Zentrifugierungsschritt ein Bakterienkonzentrat gewonnen, welches konserviert und eingelagert wird.

Gemäß einer bevorzugten Ausführungsform ist vorgesehen, dass das in Schritt a) entstehende Homogenisat entweder unmittelbar einer Dichtegradientzentrifugation unterworfen wird oder zunächst in einem für die Dichtegradientzentrifugation geeigneten Lösungsmittel aufpipettiert und anschließend einer Dichtegradientzentrifugation unterworfen wird.

Im letzteren Fall wird das in Schritt a) entstehende Homogenisat mit einem für die Dichtegradientzentrifugation geeigneten Lösungsmittel aufpipettiert und das aufpipettierte Homogenisat einer weiteren Homogenisierung unterworfen. Derartigen Lösungsmittel sind im Handel erhältlich und werden derzeit von der Firma Progen als Nycodenz®-Lösung vertrieben.

In weiterer Ausgestaltung der Erfindung ist vorgesehen, dass das in Schritt a) entstehende oder nach der Aufpipettierung erneut homogenisierte Homogenisat einer Dichtegradientzentrifugation unterworfen wird, bis das Homogenisat in Form von mehreren, insbesondere sechs Phasen fraktioniert ist, dass die festen Phasen des zentrifugierten Homogenisats, die eine hohe Konzentration an Bakterien enthalten, abgenommen werden und dass die festen Phasen als Bakterienkonzentrat unmittelbar konserviert und eingelagert oder aus den festen Phasen ein Bakterienkonzentrat gewonnen und konserviert und eingelagert wird.

Bei dieser Ausführungsform wird das entstehende Homogenisat somit einer Dichtegradientzentrifugation unterworfen, so dass das Homogenisat in Form von mehreren, insbesondere von sechs Phasen fraktioniert wird. Von diesen werden die festen Phasen abgenommen und unmittelbar oder nach Durchführung weiterer Schritte konserviert und eingelagert. Insbesondere können die beiden mittleren festen Phasen des zentrifugierten Homogenisats abgenommen werden.

Aus den abgenommenen festen Phasen kann das Bakterienkonzentrat gewonnen werden, indem
- die festen Phasen in einer physiologischen Pufferlösung, insbesondere einer physiologischen Kochsalzlösung, in Suspension gebracht werden,
- die Suspension inbesondere auf Eis inkubiert wird,
- die inkubierte Suspension einer erneuten Zentrifugation unterworfen wird und
- der bei der erneuten Zentrifugation entstehende Bodensatz als Bakterienkonzentrat konserviert und eingelagert wird.

Die Konservierung des Bodensatzes, d.h. des Bakterienkonzentrats, kann mittels Lyophilisierung erfolgen. Hierzu wird der Bodensatz zweckmäßigerweise auf einen Trägerstoff - beispielsweise auf Magermilchpulver - pipettiert und in einer Gefriertrocknungsanlage lyophilisiert. Konkret kann zur Lyophilisierung je 10 µl des Bodensatzes auf 1 ml Magermilchpulver pipettiert und in einer Gefriertrocknungsanlage lyophilisiert werden. Die anschließende Lagerung des Lyophilisats erfolgt bei wenigstens -15°C, insbesondere bei -20° C, d.h. bei einer Temperatur, welche eine Lagerung über einen langen Zeitraum bei weitestgehendem Erhalt der Keim-Lebensfähigkeit ermöglicht.

Zur Verabreichung des Bakterienkonzentrats an einen Patienten kann das Lyophilisat zur oralen Anwendung in Kapseln verfüllt werden. Alternativ ist es möglich, das Lyophilisat mit Flüssigkeit zu resuspendieren, um als Suspension einem Patienten per Einlauf oder endoskopisch verabreicht zu werden.

Bei dem erfindungsgemäßen Verfahren sind noch weitere Reinigungsschritte möglich. In diesen Reinigungsschritten werden insbesondere mittels immunomagnetischer Separation spezifisch potentiell pathogene Mikroorganismen aus dem gewonnenen und behandelten Material entfernt.

Beispielsweise kann im Rahmen der Durchführung des Verfahrens der Stuhl eines Spenders in einer physiologischen Kochsalzlösung homogenisiert werden. Dabei wird jeweils 1 g Stuhl in 8-10 ml, insbesondere 9 ml steriler physiologischer Kochsalzlösung homogenisiert. In dem nachfolgenden Schritt werden 10,5 ml des entstehenden Homogenisats auf 3,5 ml einer 80%igen Nycodenz-Lösung als Lösungsmittel in ein 15 ml-Zentrifugenröhrchen aufpipettiert. Die anschließende Dichtegradienzentrifugation erfolgt bei 4.700 x g über 80 Minuten, so dass das Homogenisat in sechs Phasen fraktioniert wird. Von diesen werden die beiden mittleren festen Phasen abgenommen und in 4 ml steriler physiologischer Kochsalzlösung eingebracht. Die so entstehende Suspension wird für 5 Minuten auf Eis inkubiert. Anschließend erfolgt eine Zentrifugation wiederrum bei 4.700 x g über eine Zeitdauer von 25 Minuten.

## Patentansprüche

1. Verfahren zur Einlagerung der Bakterienflora des Darms eines Lebewesens, insbesondere eines Menschen, bei welchem die folgenden Schritte durchgeführt werden:
a) der Stuhl eines Spenders wird in einer physiologischen Pufferlösung, insbesondere einer physiologischen Kochsalzlösung, homogenisiert,
b) aus dem entstehenden Homogenisat wird durch wenigstens einen Filtrationsschritt und/oder wenigstens einen Zentrifugierungsschritt ein Bakterienkonzentrat gewonnen und
c) das Bakterienkonzentrat wird konserviert und eingelagert.

2. Verfahren nach Anspruch 1, **dadurch gekennzeichnet, dass** das in Schritt a) entstehende Homogenisat mit einem für die Dichtegradientzentrifugation geeigneten Lösungsmittel aufpipettiert und das aufpipettierte Homogenisat einer weiteren Homogenisierung unterworfen wird.

3. Verfahren nach Anspruch 2, **dadurch gekennzeichnet, dass** 10,5 ml des in Schritt a) entstehenden Homogenisats auf 3,5 ml einer 80%igen Nycodenz-Lösung als Lösungsmittel insbesondere in einem 15 ml-Zentrifugenröhrchen aufpipettiert wird.

4. Verfahren nach einem der Ansprüche 1 bis 3, **dadurch gekennzeichnet, dass** das in Schritt a) entstehende oder nach der Aufpipettierung erneut homogenisierte Homogenisat einer Dichtegradientzentrifugation unterworfen wird, bis das Homogenisat in Form von mehreren, insbesondere sechs Phasen fraktioniert ist und die festen Phasen des zentrifugierten Homogenisats, die eine hohe Konzentration an Bakterien enthalten, abgenommen und die festen Phasen als Bakterienkonzentrat unmittelbar konserviert und eingelagert oder aus den festen Phasen ein Bakterienkonzentrat gewonnen und konserviert und eingelagert wird.

5. Verfahren nach Anspruch 4, **dadurch gekennzeichnet, dass** die Dichtegradientzentrifugation bei 4.700 x g über 80 Minuten erfolgt, wobei g die Erdbeschleunigung ist.

6. Verfahren nach Anspruch 4 oder 5, **dadurch gekennzeichnet, dass** die beiden mittleren festen Phasen des zentrifugierten Homogenisats abgenommen werden.

7. Verfahren nach einem der Ansprüche 4 bis 6, **dadurch gekennzeichnet, dass** aus den abgenommenen festen Phasen das Bakterienkonzentrat gewonnen wird, indem
- die festen Phasen in einer physiologischen Pufferlösung, insbesondere einer physiologischen Kochsalzlösung, in Suspension gebracht werden,
- die Suspension insbesondere auf Eis inkubiert wird,
- die inkubierte Suspension einer erneuten Zentrifugation unterworfen wird und
- der bei der erneuten Zentrifugation entstehende Bodensatz als Bakterienkonzentrat konserviert und eingelagert wird.

8. Verfahren nach Anspruch 7, **dadurch gekennzeichnet, dass** die beiden mittleren Phasen in 4 ml steriler physiologischer Kochsalzlösung in Suspension gebracht werden.

9. Verfahren nach Anspruch 7 oder 8, **dadurch gekennzeichnet, dass** die Suspension für 5 Minuten auf Eis inkubiert wird.

10. Verfahren nach einem der Ansprüche 7 bis 9, **dadurch gekennzeichnet, dass** die inkubierte Suspension bei 4.700 x g über eine Zeitdauer von 25 Minuten der Zentrifugation unterworfen wird.

11. Verfahren nach einem der vorherigen Ansprüche, **dadurch gekennzeichnet, dass** die Konservierung des Bodensatzes mittels Lyophilisierung erfolgt.

12. Verfahren nach Anspruch 11, **dadurch gekennzeichnet, dass** zur Lyophilisierung der Bodensatz auf einem Trägerstoff, insbesondere auf Magermilchpulver pipettiert und in einer Gefriertrocknungsanlage lyophilisiert wird.

13. Verfahren nach Anspruch 12, **dadurch gekennzeichnet, dass** zur Lyophilisierung je 10 µl des Bodensatzes auf 1 ml Magermilchpulver pipettiert und in einer Gefriertrocknungsanlage lyophilisiert wird.

14. Verfahren nach einem der vorherigen Ansprüche, **dadurch gekennzeichnet, dass** zwischen den Schritten a) und c) wenigstens ein Reinigungsschritt, insbesondere mehrere Reinigungsschritte erfolgen.

15. Verfahren nach Anspruch 14, **dadurch gekennzeichnet, dass** in wenigstens einem weiteren Reinigungsschritt insbesondere mittels immunomagnetischer Separation spezifisch potentiell pathogene Mikroorganismen aus dem Material entfernt werden.
